# EUROPEAN PATENT APPLICATION

(11) **EP 2 050 436 A1**
(43) Date of publication of application: **22.04.2009**
(21) Application number: 08016816.4
(22) Date of filing: 25.09.2008
(51) Int. Cl.: A61K 9/14, A61K 9/20, A61K 31/58

(54) **Pharmaceutical composition containing dutasteride**

(30) Priority: 21.10.2007 EP 07024952
(71) Applicant: Siegfried Generics International AG, 4800 Zofingen (CH)
(72) Inventor: Bueb, Wattraud, 4663 Aarburg (CH); Röhrich, Lambert Tillmann, 4310 Rheinfelden (CH); Steiner, Roland, 6403 Küssnacht am Rigi (CH)
(74) Representative: Braun, André jr.

(57) **Abstract**

Pharmaceutical solid composition for oral administration containing dutasteride, which comprises dutasteride in powder form, optionally in combination with one or more excipients, wherein the lower limit of the average particle size of dutasteride is higher than 2.0 µm (>2.0 micron) and the upper limit of the average particle size of dutasteride is at about 10 µm (10 micron); method of making said composition and use of the composition in the treatment of benign prostatic hyperblasia (BPH) and alopecia.

## Description

### Technical Field of the Invention

The present invention relates to a pharmaceutical composition containing dutasteride for oral administration made from an immediate release formulation containing as the active ingredient the pharmaceutically active compound Dutasteride. The pharmaceutical composition represents a solid and stable pharmaceutical dosage form, with immediate release characteristics with respect to the active ingredient.

### State of the Art

Dutasteride is inter alia known to inhibit the transformation of testosterone into 5 alpha-dihydrotestosterone (DHT) as disclosed in EP 155 096. DHT is the androgen primarily responsible for the initial development and subsequent enlargement of the prostate gland. Dutasteride is registered and indicated for the treatment of prostate gland enlargement. Dutasteride, as disclosed in EP 719 278, is also known to block both the type 1 and type 2 isoforms of the enzyme 5 alpha reductase. Dutasteride is the INN name given to the compound chemically named as 17β-[N-[2,5-bis(trifluoromethyl)phenyl]-carbamoyl]-4-aza-5α-androst-1-en-3-one, corresponding to the chemical formula (I): 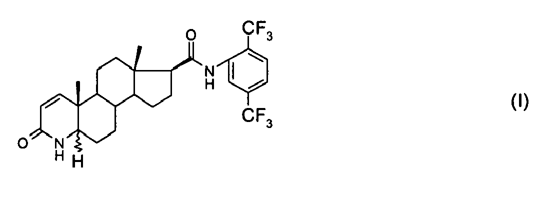 Two crystal forms of dutasteride, i.e. crystal form I and crystal form II, as well as an amorphous form, have been disclosed in US 7 022 854 B2.

Dutasteride dissolved in a mixture of mono-diglycerides of caprylic/capric acid and butylated hydroxytoluene is commercially available in the dosage form of soft gelatin capsules, which generally contain 0.5 mg of active substance. Details of this capsule are disclosed in WO 99/08684 where it is also stated that the claimed composition has an improved bioavailability over tablets or suspensions. The increased bioavailability of a solution of dutasteride supplied in a capsule over a solid composition is explained by the low solubility of the active ingredient in many organic solvents whereby it is further mentioned that these solubility challenges can result in reduced or unpredictable bioavailability. According to the state of the art, dutasteride in the form of a solution is the only dosage form with a satisfactory bioavailability as required for an immediate release formulation. Dutasteride in fact is exclusively marketed as a solution of the active ingredient contained in a soft gelatin capsule.

It is generally known that a capsule filled with a solution usually has a limited stability and a relatively short shelf life period. In the Study No. ARI10018 it was found that in earlier conventional dissolution testing of GI 198745 (dutasteride) soft gelatin capsules, a problem with cross-linking of gelatin was discovered, resulting in a decreased solubility of the gelatin and a reduced rate of drug release during in vitro testing and that these properties can reduce the stability of the dosage form, in particular under tropic conditions with elevated temperature and humidity. Furthermore the manufacture of capsules containing a solution involves more complex processes than the manufacture of a solid form, in particular of a tablet.

In US-A-2006/0204588 a solid nanoparticulate composition comprising finasteride, dutasteride, tamsulosin hydrochloride, or a combination thereof is described, wherein the particles have an effective average particle size of less than two microns (<2µm). Methods of preparing an active ingredient of an average particle size of less than two microns are demanding in terms of special mechanical equipment required and handling of the dusty particles. Further, according to the mentioned reference the presence of auxiliary substances stabilizing the specific surface and particle size of the active ingredient is required.

### Summary of the Invention

It has now been found that immediate release compositions in solid form containing dutasteride as active pharmaceutical ingredient can be made which have satisfactory characteristics, especially an improved stability on storage and on further processing into solid dosage forms, by using conventional pharmaceutical technologies and pharmaceutical equipment. These solid immediate release compositions have a fast dissolution rate, and can be produced with known straight forward processes, such as conventional crystallization techniques, optionally followed by a milling process, whereby these processes can be carried out in the presence or absence of conventional additives which will be contained in the final composition. The final composition does not need to contain special additives such as surface stabilizers.

The processes of preparing immediate release compositions in solid form containing dutasteride as active pharmaceutical ingredient according to the present invention are easy to perform, allow considerable savings and offer distinct advantages over the preparation of such prior art compositions, such as nanoparticulate compositions.

The solid dosage forms may contain 0.05 mg to 2.0 mg of dutasteride per dosage unit containing dutasteride in crystalline or amorphous form. According to the present invention the lower limit of the average particle size of dutasteride is higher than 2.0 µm (>2.0 micron); the upper limit of the average particle size of dutasteride is about 10 µm (10 micron).

The expression "pharmaceutical composition" containing dutasteride, as used herein, means the final solid dosage form as defined according to the present invention, for example a tablet, or a granulate which is enclosed in a hard gelatin capsule. The tablet or the granules, for example, may be coated.

The expression "intermediate formulation" as used herein, means the intermediate mixture which is used to produce the final "pharmaceutical composition". The intermediate formulation may be present for example in the form of a powdery composition comprising dutasteride in powder form, optionally in combination with one or more excipients, wherein the lower limit of the average particle size of dutasteride is higher than 2.0 µm (>2.0 micron) and the upper limit of the average particle size of dutasteride is at about 10 µm (10 micron) and preferably lower than 10 µm (10 micron).

### Detailed Description of the Invention

The present invention is defined in the claims. The present invention specifically refers to a pharmaceutical composition containing dutasteride as made from an intermediate formulation containing as the active ingredient the pharmaceutically active compound dutasteride and optionally further additives, characterized in that said pharmaceutical composition is a solid composition for oral administration and that said intermediate formulation comprises dutasteride in powder form, optionally in combination with one or more excipients, wherein the lower limit of the average particle size of dutasteride is higher than 2.0 µm (>2.0 micron) and the upper limit of the average particle size of dutasteride is at about 10 µm (10 micron).

The present invention also refers to a method of producing the pharmaceutical composition as defined in the present invention comprising the steps of providing dutasteride powder with an average particle size having a lower particle size limit that is higher than 2.0 µm (>2.0 micron) and an upper particle size limit that is at about 10 µm (10 micron), mixing said dutasteride powder with excipients in selected amounts and further processing of the obtained intermediate formulation into the desired end product.

The present invention further refers to the use of the intermediate formulation containing the active ingredient for the production of a pharmaceutical composition, as a dosage form, for the treatment of benign prostatic hyperblasia (BPH) or alopecia (hairloss). The present invention also relates to the use of the pharmaceutical composition as defined in the present invention in the treatment of benign prostatic hyperblasia (BPH).

The present invention further refers to treating human patients in need of such treatment with a composition of the present invention in particular in the treatment of benign prostatic hyperblasia (BPH) and in the treatment of alopecia (hairloss).

The pharmaceutical composition of the present invention comprises dutasteride as active pharmaceutical ingredient in amounts of about 0.05 mg to 2.0 mg per dosage unit, preferably 0.1 to 1.5 mg, and for example specifically in 0.05 mg, 0.10 mg, 0.20 mg, 0.50 mg, 0.80 mg, 1.00 mg, 1.50 mg, or 2.0 mg per dosage unit. The active ingredient dutasteride may be present in a crystalline form or amorphous form, preferably in crystal form I or crystal form II, preferably crystal form I.

The pharmaceutical composition is a solid composition for oral administration and is made from the intermediate formulation. Both comprise dutasteride in powder form, optionally in combination with one or more excipients, wherein the lower and the upper limit of the average particle size of dutasteride is as defined above.

The lower limit of the average particle size of dutasteride is higher than 2.0 µm (>2.0 micron), preferably at about 2.1 µm (2.1 micron), preferably at about 2.2 µm (2.2 micron), preferably at about 2.3 µm (2.3 micron), preferably at about 2.4 µm (2.4 micron) and preferably at about 2.5 µm (2.5 micron).

The upper limit of the average particle size of dutasteride is at about 10 µm (10 micron), preferably lower than 10 µm (10 micron), preferably at about 9 µm (9 micron), preferably at about 8 µm (8 micron), preferably at about 7 µm (7 micron), preferably at about 6 µm (6 micron).

Preferably the average particle size of dutasteride is within the range of higher than 2.0 µm (>2.0 micron) to 10 µm (10 micron), preferably within the range of 2.1 µm (2.1 micron) to 10 µm (10 micron), preferably within the range of 2.2 µm (2.2 micron) to 9 µm (9 micron), preferably within the range of 2.3 µm (2.3 micron) to 8 µm (8 micron), preferably within the range of 2.4 µm (2.4 micron) to 7 µm (7 micron), preferably within the range of 2.5 µm (2.5 micron) to 6 µm (6 micron). Preferably 50%, preferably 60% and preferably 70% of the powdery particles of dutasteride are within the indicated average particle size values as given above.

In order to produce these particle sizes any process available may be applied such as crystallization, mechanical processes or dissolution or melting/dispersion methods. Such processes are conventional and are known per se. Preferred is a crystallization process optionally followed by a milling process, e.g. milling in a jet-mill until the required average grain size distribution has been obtained.

In addition to dutasteride, preferably at least one of each of the following three types of pharmaceutically acceptable excipients is present in the composition of the present invention: (a) a filling agent, (b) a binding agent, and/or (c) a glidant/lubricant. Such compounds are known per se.

The pharmaceutical composition according to the present invention may additionally comprise one or more optional excipients of the groups comprising: (d) surfactants and (e) disintegrants.

Optionally the pharmaceutical composition of the present invention is coated with a film, comprising as excipients at least one compound of a group not already listed above, selected from: (f) film forming agents, and/or (g) softeners, and/or (h) dyes, and/or (i) other compounds known per se as film forming agents or corresponding additives, including glidants/lubricants. Excipients of groups (f) to (i) are typically available as ready-to-use mixtures.

As excipients belonging to the classes mentioned above, many generally acceptable substances are known and are commercially available. Said substances are well known to the expert in the art. The list of substances given below includes as examples typical excipients of the various classes. Their amount in % by weight, based on the total weight of the composition may vary within the ranges given.

Filling agents [component (a)] are for example calcium carbonate, cyclodextrin, dibasic calcium phosphate, tribasic calcium phosphate, calcium sulfate, calcium silicate, magnesium carbonate, magnesium trisilicate, microcrystalline cellulose, powdered cellulose, maltodextrin, dextrose, fructose, lactose, L-HPC, preferably as monohydrate, starch, pregelatinated starch, mannitol, maltol, maltitol, sorbitol, sucrose, xylitol, new composite compounds such as Micro-cellac®, Cellactose®, Ludipress®, Prosolv®, and related compounds. Said filling agent may be present in an amount from 1% to 99%, preferably 20% to 97%, preferably 40% to 95%, based on the total weight of the composition.

Binding agents [component (b)] are for example acacia, carboxymethylcellulose, dextrose, fructose, gelatin, hydroxypropylcellulose, hydroxypropylmethylcellulose, maltodextrin, maltol, maltitol, mannitol, polyethylenglycol, povidone, hydroxypovidone, polyvinylalcohol, alginate, sorbitol, starch, pregelatinized starch, sucrose, traganth, xanthan gum, xylitol, and related compounds. Said binding agent may be present in an amount from 0% to up to 30%, preferably 0% to up to 25%, preferably from 1% to 20%, based on the total weight of the composition.

Glidants/lubricants [component (c)] are for example calcium stearate, magnesium stearate, zinc stearate, sodium stearyl-fumarat, glycerin monostearat, glycerol palmitostearate, glycerol monooleate, glyceryl behenate, cetostearyl alcohol, castor oil hydrogenated, stearyl alcohol, colloidal silicon dioxide, docusate sodium, lecithin, polyethylenglycol, potassium benzoate, sorbitan esters, stearyl alcohol, talc, vegetable oil hydrogenated, wax, and related compounds. Said glidants/lubricants may be present in an amount from 0% to up to 5%, preferably from 0.1% to 3.5%, preferably from 0.5% to 2.5%, based on the total weight of the composition.

Surfactants [component (d)] are for example docusate sodium, lecithin, polyoxyethylene alkyl ether, polyoxyethylene sorbitan fatty acid ester, polyoxyethylene stearates, sorbitan esters, stearic acid, and related compounds. Said surfactants may be present in an amount from 0% to up to 10%, preferably from 0.01% to 5%, preferably from 0.015 to 2%, based on the total weight of the composition.

Disintegrants [component (e)] are for example microcrystalline cellulose, colloidal silicon dioxide, calcium silicate, cros-carmellose sodium, carmellose calcium, crospovidone, L-HPC, sodium starch glycollate, starch, pregelatinized starch, and related compounds. Said disintegrants may be present in an amount from 0% to up to 20%, preferably from 1% to 15%, and preferably from 2% to 12%, based on the total weight of the composition.

The film of a coated composition of the invention comprises the following excipients [component (f)] to [component (i) as mentioned above] in a total amount of 0% to up to 10%, preferably from 0.10% to 7%, and preferably from 0.2% to 5%, by weight of the coating composition

Film forming substances [component (f)] are e.g acacia, alginic acid, carboxymethylcellulose, carrageenan, ethylcellulose, hydroxyethylcellulose, hydroxyl propylmethylcellulose, methylcellulose, polyvinyl alcohol, polymethacrylates, povidone, shellac, and related compounds.

Gliding agents/lubricants may also be used as components of the film and are the same as given above for [component (c)].

Softeners [component (g)] are for example acetyltributyl citrate, acetyltriethyl citrate, dibutyl phthalate, dibutyl sebacetate, diethyl phthalate, dimethyl phthalate, glyceryl monosteararate, glyceryl palmitostearate, glyceryl monooleate, lecithin, medium chain triglycerides, glycerin, polyethyleneglycol, propylenglycol, sorbitol, triacetin, tributyl citrate, triethyl citrate, wax, and related compounds.

Dyes [component (h)] are for example coloring agents such as titanium dioxide, ferric oxide, and other inorganic oxides and related compounds.

Other compounds [component (i)] are e.g. simethicone, lactose, mannitol, cellulose, stearyl alcohol, stearic acid, sorbitan ester, docussate sodium, and related compounds.

The selection and use of the above mentioned excipients is not limited to the substances listed as examples. It is understood that any given excipient may serve more than one function, e.g. as binder and filling agent. This is known to the expert in the art.

The pharmaceutical composition containing dutasteride, i.e. the final solid dosage form, can be present as a pharmaceutical dosage form known per se, preferably as a powder, as pellets, as a granulate, as micro-spheres, as micro-tablets, as a tablet, or filled in a capsule, for example in a hard gelatin capsule. A tablet or capsule may vary in shape and may be for example round, oblong, oval, polyedric or be present in any other shape.

The intermediate formulation which is used to produce the pharmaceutical composition preferably is present for example in the form of a powder comprising dutasteride as defined above, or as a powder mixture comprising dutasteride as defined above in combination with one or more excipients. Of course, not only the pharmaceutical composition but also the intermediate formulation may be administered to a human subject for treatment.

A preferred embodiment of the present invention is a pharmaceutical composition as a dosage form in the form of an immediate release tablet. The tablet may optionally be coated. Each tablet contains 0.05 to 2.0 mg of dutasteride, preferably of crystal form I, and excipients, in amounts given above. Typically such a tablet releases at least 80% of the active ingredient dutasteride within 30 minutes, preferably within 20 minutes. These values are comparable with the dissolution rate of the commercially available soft gelatin capsule.

The invention furthermore includes processes for preparing the pharmaceutical composition containing dutasteride, i.e. the final solid dosage form, as well as the intermediate formulation for preparing the pharmaceutical composition. The individual steps of these processes are performed on standard equipment known in pharmaceutical technology.

Optionally dutasteride is brought into the form having the average particle size distribution as mentioned above followed by mixing the dutasteride powder with any required excipient. The processes and the analytical determination of the particle size are carried out in standard equipment, as used in the manufacture of active pharmaceutical ingredients.

Dry or wet standard processes are available for mixing dutasteride with excipients. The process for preparing compositions in form of a dry powder blend comprises mixing dutasteride with above mentioned excipients, for example in a low-shear mixer or a container blender. Sieving steps can be optionally included in the process to increase homogeneity of the powder blend. Granules can be prepared by dry granulation, e.g. roller compaction. In a wet mixing process the active pharmaceutical ingredient and the excipients are mixed with a liquid to form a suspension, which is converted into granulates by drying. Wet granulation with water or with an organic solvent is typically carried out in a high shear mixer, in a fluid bed granulator or in any other established equipment.

The pharmaceutical composition in the form of a powder blend or granules can be filled either in capsules or sachets or can be compressed into tablets on a tablet press, preferably on a rotary tablet press. Optionally some of the excipients as mentioned above may be added in order to improve the manufacturing process or formulation characteristics.

Optionally dosage forms such as granules, pellets, micro-spheres, micro-tablets, tablets and also filled capsules may be coated by standard coating processes in suitable equipment.

The compositions of the invention have been tested and characterized by established procedures for the analysis of pharmaceutical products, including a method for the determination of dissolution rates according to Pharm. Eur. and USP. The active ingredient of an immediate release composition is known do dissolve almost completely within 30 minutes or less.

The present invention refers to a method of producing the pharmaceutical composition as defined in the present invention, said method comprising the steps of providing dutasteride with an average particle size distribution within the range of higher than 2.0 µm (>2.0 micron) to 10 µm (10 micron), with the preferred ranges as mentioned above, optionally in the presence of one or more excipients, mixing the dutasteride with the optional excipients in selected amounts and subsequently processing the obtained mixture, i.e. the obtained intermediate formulation, into the desired end product, i.e. the pharmaceutical composition.

As a result of intensive process research, it has been possible to define processes for preparing carefully balanced mixtures of dutasteride with one or more excipients, followed by converting the resulting intermediates into pharmaceutical immediate release compositions. The advantageous characteristics of these compositions have been determined by the above-mentioned analytical procedures. The compositions of the invention therefore exhibit the desired characteristics regarding immediate release, stability and simplicity of the manufacturing process. The dosage forms of the invention and in particular the tablets are stable both to the production process and during storage in conventional packaging, such as blister packs, typically for a shelf life of 2 years, or even 3 to 5 years. In particular these immediate release compositions offer advantages in terms of shelf life in tropic conditions.

The dosage forms of the invention are useful for the human indications of dutasteride. Preferably the dosage forms disclosed herein are indicated for the treatment of prostate gland enlargement (BPH) and of alopecia. Together with further advantages in terms of satisfactory content uniformity, good stability, long shelf life and simplicity of the processes the compositions of the invention offer considerable advantages over the state of the art. The details of the invention are given in the examples provided below, which are given for illustrations only and therefore should not be construed to limit the scope of the present invention.

### Examples

### (1.) Preparation of dutasteride powder

100 g of crude dutasteride, prepared according to EP 019 278, Examples 1 and 2, is purified by recrystallization from methanol/acetonitril to yield pure, crystalline dutasteride. The purity is not less than 98 %, as determined by HPLC. Milling in a jet-mill MC 50 provides a white to off white colored powder with an average particle size of 2.5 micron (µm), as determined by means of a laser diffraction sensor (Helos, supplier Sympatec GmbH).

In a laboratory type blending equipment (e.g. Turbula blender) dutasteride, as prepared in (1.) above, is diluted gradually by addition and mixing of the excipients of the inner phase, in amounts according to Tables 1 to 3. The resulting blend is sieved through standard sieves (e.g. 0.5mm) for further improvement of homogenous API distribution. The mixture obtained is named the inner phase (see Tables). The lubricant and optionally the disintegrant are added finally as components of the outer phase and the mixture is blended for another two minutes. This final blend is compressed on a rotary tabletting machine to obtain immediate release tablets. The components as listed in the following Tables 1-8 are given per dosage unit.

### Example 1: Tablet Formulation (0.5 mg in 150 mg tablet)

**Table 1**

| | Function | Amount per unit (mg) |
|---|---|---|
| Inner Phase: | | |
| Dutasterid | Active ingredient | 0.5 |
| Lactose monohydrate | Filling agent | 90 |
| Microcrist. cellulose | Filling agent | 47.7 |
| Povidone | Binder | 4.5 |
| Sodium dodecylsulfate | Surfactant | 0.3 |
| Sodium starch glycollate | Disintegrant | 6.0 |
| Outer Phase: | | |
| Magnesium stearate | Lubricant | 1.0 |
| Total weight | | 150 |

### Example 2: Tablet Formulation (150 mg tablet)

**Table 2**

| | Function | Amount per unit (mg) |
|---|---|---|
| Inner Phase: | | |
| Dutasterid | Active ingredient | 0.5 |
| Lactose monohydrate | Filling agent | 79.9 |
| Microcrist. cellulose | Filling agent | 40 |
| Pregelatinized starch | Binder | 22.6 |
| Sodium dodecylsulfate | Surfactant | 0.2 |
| Sodium starch glycollate | Disintegrant | 6.0 |
| Outer Phase: | | |
| Magnesium stearate | Lubricant | 0.8 |
| Total weight | | 150 |

### Example 3: Tablet Formulation (150 mg tablet)

**Table 3**

| | Function | Amount per unit (mg) |
|---|---|---|
| Inner Phase: | | |
| Dutasterid | Active ingredient | 0.5 |
| Lactose monohydrate | Filling agent | 100 |
| Microcrist. cellulose | Filling agent | 41.5 |
| Sodium dodecylsulfate | Surfactant | 1.0 |
| Sodium starch glycollate | Disintegrant | 6.0 |
| Outer Phase: | | |
| Magnesium stearate | Lubricant | 1.0 |
| Total weight | | 150 |

The rate of dissolution of the resulting tablets was measured according to the following method.

| Dissolution method | |
|---|---|
| Apparatus | Ph. Eur. (current edition, paddle apparatus), USP (current edition, apparatus 2) |
| Temperature | 37°C ± 0.5°C |
| Volume dissolution medium | 900 ml |
| Stirring speed | 50 rpm |
| Sampling time | 10 min., 20 min., 30 min. |
| Dissolution medium | 0.1 N HCl with 2% (w/v) sodium dodecyl sulfate |

As a typical result of the compositions of the invention more than 80% of dutasteride dissolved within 10 minutes.

### Examples for Wet Granulation (Water)

In a laboratory type high shear mixer(e.g.Diosna) dutasteride, prepared according to (1.) above, is diluted gradually by addition and mixing of the excipients of the inner phase, in amounts according to Tables 4 and 5. The resulting powder blend is granulated in the high shear mixer by addition of water. The wet mass obtained is dried on trays in a hurdle dryer at 40 - 50°C and the resulting dry mass is sieved through a 1.0 mm screen. The granules obtained are named the inner phase (see Tables). The components of the outer phase are added and the mixture is blended for two minutes in a laboratory type blending equipment (e.g. Turbula blender). This final blend is compressed on a rotary tabletting machine to obtain immediate release tablets.

### Example 4: Tablet Formulation (0.5 mg in 150 mg tablet)

**Table 4**

| | Function | Amount per unit (mg) |
|---|---|---|
| Inner Phase: | | |
| Dutasterid | Active ingredient | 0.5 |
| Lactose monohydrate | Filling agent | 90 |
| Microcrist. cellulose | Filling agent | 47.7 |
| Povidone | Binder | 4.5 |
| Sodium dodecylsulfate | Surfactant | 0.3 |
| Purified water | Solvent | q.s. |
| Outer Phase: | | |
| Sodium starch glycollate | Disintegrant | 6.0 |
| Magnesium stearate | Lubricant | 1.0 |
| Total weight | | 150 |

### Example 5: Tablet Formulation (150 mg tablet)

**Table 5**

| | Function | Amount per unit (mg) |
|---|---|---|
| Inner Phase: | | |
| Dutasterid | Active ingredient | 0.5 |
| Lactose monohydrate | Filling agent | 79.8 |
| Microcrist. cellulose | Filling agent | 40 |
| Pregelatinized starch | Binder | 22.6 |
| Sodium dodecylsulfate | Surfactant | 0.3 |
| Purified water | Solvent | q.s. |
| Outer Phase: | | |
| Sodium starch glycollate | Disintegrant | 6.0 |
| Magnesium stearate | Lubricant | 0.8 |
| Total weight | | 150 |

In a dissolution test performed according to the paddle method described above, more than 80 % of dutasteride dissolved within 10 minutes.

### Examples for Wet Granulation (API dissolved in ethanol)

Dutasteride, prepared according to (1.) above, is dissolved in Ethanol 96%. The remaining components (inactive ingredients) of the inner phase are mixed separately in a high shear mixer (e.g. Diosna) until homogeneity is reached. The resulting powder blend is then granulated in the high shear mixer by addition of the ethanolic solution of dutasteride. The resulting wet granulate (inner phase) is processed further, as described above for granulation with water, to obtain immediate release tablets. Components used are given in Tables 6 and 7.

### Example 6: Tablet Formulation (0.5 mg in 150 mg tablet)

**Table 6**

| | Function | Amount per unit (mg) |
|---|---|---|
| Inner Phase: | | |
| Dutasterid | Active ingredient | 0.5 |
| Lactose monohydrate | Filling agent | 90 |
| Microcrist. cellulose | Filling agent | 47.7 |
| Povidone | Binder | 4.5 |
| Sodium dodecylsulfate | Surfactant | 0.3 |
| Ethanol 96% | Solvent | q.s. |
| Outer Phase: | | |
| Sodium starch glycollate | Disintegrant | 6.0 |
| Magnesium stearate | Lubricant | 1.0 |
| Total weight | | 150 |

### Example 7: Tablet Formulation (150 mg tablet)

**Table 7**

| | Function | Amount per unit (mg) |
|---|---|---|
| Inner Phase: | | |
| Dutasterid | Active ingredient | 0.5 |
| Lactose monohydrate | Filling agent | 79.8 |
| Microcrist. cellulose | Filling agent | 40 |
| Pregelatinized starch | Binder | 22.6 |
| Sodium dodecylsulfate | Surfactant | 0.3 |
| Ethanol 96% | Solvent | q.s. |
| Outer Phase: | | |
| Sodium starch glycollate | Disintegrant | 6.0 |
| Magnesium stearate | Lubricant | 0.8 |
| Total weight | | 150 |

In a dissolution test performed according to the paddle method described above, more than 85 % of dutasteride dissolved within 10 minutes.

### Examples for Hot Melt Granulation (API dissolved in PEG 6000)

Dutasteride is diluted stepwise with the excipients as given in Table 8 (for the inner phase), by several blending and sieving steps. The obtained powder blend is granulated in a high shear mixer by heating to 70 - 75°C whereby the melted mass is diluted stepwise with calcium silicate. After cooling, the dry mass is sieved through a 1.0 mm screen. Finally magnesium stearate is added and the mixture is blended for two minutes. The obtained blend is compressed on a rotary tabletting machine to obtain immediate release tablets.

### Example 8: Tablet Formulation (0.5 mg in 300 mg tablet)

**Table 8**

| | Function | Amount per unit (mg) |
|---|---|---|
| Inner Phase: | | |
| Dutasterid | Active ingredient | 0.5 |
| PEG 6000 | Solvent / Filling agent | 182.7 |
| Microcristalline cellulose | Filling agent | 47.0 |
| Sodium dodecylsulfate | Surfactant | 0.2 |
| Sodium starch glycollate | Disintegrant | 12.0 |
| Croscarmellose sodium | Disintegrant | 22.0 |
| Outer Phase: | | |
| Calcium silicate | Filling agent | 34.0 |
| Magnesium stearate | Lubricant | 1.6 |
| Total weight | | 300 |

In a dissolution test performed according to the paddle method described above, more than 85 % of dutasteride dissolved within 10 minutes.

## Claims

1. Pharmaceutical composition containing dutasteride as made from an intermediate formulation containing as the active ingredient the pharmaceutically active compound dutasteride and optionally further additives, **characterized in that** said pharmaceutical composition is a solid composition for oral administration and that said intermediate formulation comprises dutasteride in powder form, optionally in combination with one or more excipients, wherein the lower limit of the average particle size of dutasteride is higher than 2.0 µm (>2.0 micron) and the upper limit of the average particle size of dutasteride is at about 10 µm (10 micron).

2. Composition according to claim 1, **characterized in that** the active pharmaceutical ingredient is present in amounts of 0.05 mg to 2.0 mg per dosage unit, preferably 0.1 to 1.5 mg.

3. Composition according to claim 1 or 2, **characterized in that** the active ingredient is present in a crystalline form or amorphous form, preferably in crystal form I or crystal form II, preferably as crystal form I.

4. Composition according to any one of claim 1-3, **characterized in that** the lower limit of the average particle size of dutasteride is higher than 2.0 µm (>2.0 micron), preferably at about 2.1 µm (2.1 micron), preferably at about 2.2 µm (2.2 micron), preferably at about 2.3 µm (2.3 micron), preferably at about 2.4 µm (2.4 micron) and preferably at about 2.5 µm (2.5 micron).

5. Composition according to any one of claim 1-4, **characterized in that** the upper limit of the average particle size of dutasteride is at about 10 µm (10 micron), preferably lower than 10 µm (10 micron), preferably at about 9 µm (9 micron), preferably at about 8 µm (8 micron), preferably at about 7 µm (7 micron), preferably at about 6 µm (6 micron).

6. Composition according to any one of claim 1-5, **characterized in that** the average particle size of dutasteride is within the range of higher than 2.0 µm (>2.0 micron) to 10 µm (10 micron), preferably within the range of 2.1 µm (2.1 micron) to 10 µm (10 micron), preferably within the range of 2.2 µm (2.2 micron) to 9 µm (9 micron), preferably within the range of 2.3 µm (2.3 micron) to 8 µm (8 micron), preferably within the range of 2.4 µm (2.4 micron) to 7 µm (7 micron), preferably within the range of 2.5 µm (2.5 micron) to 6 µm (6 micron) and wherein preferably 50%, preferably 60% and preferably 70% of the powdery particles of dutasteride are within these average particle size values.

7. Composition according to any one of claim 1-6, **characterized in that** at least one of each of the following types of pharmaceutically acceptable excipients is present in the composition: (a) a filling agent, (b) a binding agent, and/or (c) a gliding agent/lubricant.

8. Composition according to any one of claim 1-7, **characterized in that** additionally one or more excipients of the groups comprising: (d) surfactants and (e) disintegrants, is present.

9. Composition according to any one of claim 1-8, **characterized in that** the composition is coated with a film comprising as excipients at least one compound of the group selected from: (f) film forming agents, (g) softeners, (h) dyes and (i) other compounds known per se as film forming agents or corresponding additives, including glidants/lubricants.

10. The composition according to any one of claim 1-9, **characterized in that** the following excipients are present in amounts given in percent in weight based on the total weight of the tablet:
(a) a filling agent, present in an amount from 1 % to 99 %%, preferably 20 % to 97 %, preferably 40 % to 95 %;
(b) a binding agent, present in an amount from 0 % to up to 30 %, preferably 0 % to up to 25 %, preferably 1 % to 20 %;
(c) a glidant/ lubricant, present in an amount from 0 % to up to 5 %, preferably 0.1 % to up to 3.5 %, preferably 0.5 % to 2.5 %;
(d) a surfactant, present in an amount from 0 % to up to 10%, preferably 0.01 % to 5 %, preferably 0.01 to 2 %;
(e) a disintegrant, present in an amount from 0% to up to 20%, preferably 1 % to 15 %, preferably 2 % to 12 %.

11. A composition according to any one of claim 1-10, **characterized in that** said composition is coated with a film comprising the following excipients in a total amount in % by weight of 0 to up to 10 %, preferably 0.10 to 7 %, preferably 0.2 % to 5 %: of (f) film forming agent, (g) softener, (h) dyes, (i) others.

12. A composition according to any one of claim 1-11, **characterized in that** said composition is present as a powder, as pellets, as a granulate, as micro-spheres, as micro-tablets, as a tablet, or filled into a capsule, preferably in a hard gelatin capsule.

13. Process of producing the pharmaceutical composition according to any one of the claims 1-12, said process comprising the steps of providing dutasteride with an average particle size distribution within the range of higher than 2.0 µm (>2.0 micron) to 10 µm (10 micron), mixing the dutasteride with excipients in selected amounts and subsequently processing the obtained mixture into the desired pharmaceutical composition.

14. The process of claim 13 comprising the pharmaceutical standard processes of mixing in solid state, or alternatively dry or wet granulation to provide the immediate release formulation, and conversion of said formulation into the final composition.

15. The process of claim 14, comprising a granulation process, preferably granulation in dry form in a roller compactor or any other equipment for dry granulation, or granulation in wet form in the presence of an organic solvent or water, carried out in a high shear mixer or in a fluid bed granulator or in any other equipment for wet granulation, or for extrusion, optionally drying and/or milling of the resulting intermediate selected from the group consisting of pellets, granules or microspheres, micro-tablets, compressing of the intermediate obtained on a tabletting machine.

16. The process of claims 12 to 14, wherein the pellets, granules, microsphere, micro-tablets or tablets are additionally coated.

17. The process for the preparation of a composition according to any one of the claims 1-12 in form of a filled capsule, which comprises processing dutasteride and pharmaceutically acceptable excipients according to any one of the of claims 13-16 to obtain pellets, granules, microsphere, micro-tablets or tablets which optionally are coated and filling said pellets, granules, microsphere, micro-tablets or tablets into a capsule shell.

18. The intermediate formulation as used to produce the composition according to any one of the claims 1-12, **characterized in that** said intermediate formulation comprises dutasteride in powder form, optionally in combination with one or more excipients, wherein the lower limit of the average particle size of dutasteride is higher than 2.0 µm (>2.0 micron) and the upper limit of the average particle size of dutasteride is at about 10 µm (10 micron).

19. The use of the intermediate formulation as defined in claim 18 for the production of a pharmaceutical composition for the treatment of benign prostatic hyperblasia (BPH) or alopecia (hairloss).

20. The use of the pharmaceutical composition as defined in any one of the claims 1-12 in the treatment of benign prostatic hyperblasia (BPH) and alopecia.

21. Method of treating human patients in need of such treatment with a composition as defined in any one of claims 1-12, in particular in the treatment of benign prostatic hyperblasia (BPH) and in the treatment of alopecia (hairloss).
